(19) Europäisches Patentamt / European Patent Office / Office européen des brevets

(11) **EP 3 184 124 B1**

(12) **EUROPÄISCHE PATENTSCHRIFT**

(45) Veröffentlichungstag und Bekanntmachung des Hinweises auf die Patenterteilung:
**26.06.2019   Patentblatt 2019/26**

(51) Int Cl.:
*A61L 15/18* *(2006.01)*          *A61L 15/42* *(2006.01)*

(21) Anmeldenummer: **15201614.3**

(22) Anmeldetag: **21.12.2015**

(54) **WUNDSYSTEM ENTHALTEND ZWEI STOFFE**

WOUND SYSTEM CONTAINING TWO MATERIALS

SYSTEME DE SOIN DE BLESSURE CONTENANT DEUX SUBSTANCES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**28.06.2017   Patentblatt 2017/26**

(73) Patentinhaber: **Paul Hartmann AG**
**89522 Heidenheim (DE)**

(72) Erfinder:
• **BAUER, Jochen**
  **89522 Heidenheim (DE)**
• **SMOLA, Hans**
  **89522 Heidenheim (DE)**
• **WEISS, Pierre-Alain**
  **65824 Schwalbach am Taunus (DE)**
• **RIEDEL, Hendrik**
  **60435 Frankfurt am Main (DE)**
• **MISCHO, Horst**
  **54295 Trier (DE)**

(74) Vertreter: **Ter Meer Steinmeister & Partner**
**Patentanwälte mbB**
**Nymphenburger Straße 4**
**80335 München (DE)**

(56) Entgegenhaltungen:
WO-A1-92/11043          WO-A1-2005/079913
WO-A1-2007/068490      WO-A1-2010/000451
WO-A1-2014/178945      WO-A1-2017/083166
WO-A2-2005/004982      DE-U1- 9 310 702
US-A1- 2014 107 740

• JAIDEEP BANERJEE ET AL: "Improvement of Human Keratinocyte Migration by a Redox Active Bioelectric Dressing", PLOS ONE, Bd. 9, Nr. 3, 3. März 2014 (2014-03-03), Seite e89239, XP055273094, DOI: 10.1371/journal.pone.0089239
• CHAO YU ET AL: "Effects and mechanisms of a microcurrent dressing on skin wound healing: a review", MILITARY MEDICAL RESEARCH, BIOMED CENTRAL LTD, LONDON, UK, Bd. 1, Nr. 1, 24. November 2014 (2014-11-24), Seite 24, XP021203816, ISSN: 2054-9369, DOI: 10.1186/2054-9369-1-24
• EMILY WHITCOMB ET AL: "Demonstration of a Microcurrent-Generating Wound Care Device for Wound Healing Within a Rehabilitation Center Patient Population", JOURNAL OF THE AMERICAN COLLEGE OF CLINICAL WOUND SPECIALISTS, Bd. 4, Nr. 2, 1. Juni 2012 (2012-06-01), Seiten 32-39, XP055272359, ISSN: 2213-5103, DOI: 10.1016/j.jccw.2013.07.001

**Beschreibung**

Die Erfindung betrifft ein Wundsystem gemäß Anspruch 1, welches insbesondere in der Wundbehandlung während der Epithelisierungsphase eingesetzt wird. Das Wundsystem umfasst eine erste, einen ersten Stoff enthaltende Schicht sowie einen zweiten außerhalb der ersten Schicht angeordneten Stoff, wobei der erste und der zweite Stoff unterschiedliche Standardpotentiale aufweisen. Das beschriebene Wundsystem kann insbesondere bei der feuchten Wundbehandlung eingesetzt werden.

Die Heilung von Hautwunden beruht auf der Fähigkeit der Haut Epithel- sowie Binde- und Stützgewebe zu regenerieren. Die Regeneration selbst ist durch ein komplexes Geschehen von ineinander übergreifenden Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase).

Unter einer Wunde wird die Trennung des Zusammenhangs von Geweben der Körperhülle bei Menschen oder Tieren verstanden. Sie kann mit einem Verlust an Substanz verbunden sein.

Zur Unterstützung der einzelnen Wundheilungsphasen sind zahlreiche Vorschläge in der Literatur beschrieben. So werden beispielsweise in der WO 2010/000451 eine mehrschichtige Wundauflage sowie in der WO 2011/141454 eine Wundauflage zur Wundbehandlung beschrieben, die sich insbesondere dafür eignen, die Wunde in einem feuchten Milieu zu halten.

Weiterhin wird berichtet, dass durch das Anlegen eines elektrischen Mikrostroms die Wundheilung positiv beeinflusst werden kann. Es wird angenommen, dass der elektrische Mikrostrom oder auch nur das Anlegen eines elektrischen Feldes das Wachstum von Gewebe und Bakterien/Viren, welche unter Umständen in die Wunde gelangt sein können, beeinflusst.

In der EP 1 715 915 B1 wird eine Gewebekontaktvorrichtung beschrieben, welche dazu geeignet ist, auf ein Gewebe aufgebracht zu werden und einen elektrischen Mikrostrom zu erzeugen, der durch das angrenzende Wundgewebe fließt. Die beschriebene Gewebekontaktschicht umfasst ein Substrat sowie mehrere erste und zweite mit dem Substrat verbundene Behältnisse. Ausgewählte erste Behältnisse enthalten ein Reduktionsmittel und ausgewählte zweite Behältnisse enthalten ein Oxidationsmittel. Sowohl die Behältnisoberflächen der ersten als auch die zweiten Behältnisse sind in der Nähe einer ersten Substratoberfläche angebracht, bevorzugt sind die ersten und zweiten Behältnisse mit der Substratoberfläche verbunden. Zudem bilden die ersten Behältnisse ein erstes Muster und die zweiten Behältnisse ein zweites Muster, wobei sich die Muster durchdringen.

US2014107740 beschreibt galvanische antimikrobielle Wundauflagen mit Silber und Zink in wasserlöslichen Träger/Bindemitteln, angeordnet auf einem Basismaterial in verschiedenen Punkt- und Kreisflächen-Mustern.

Der vorliegenden Erfindung liegt die Aufgabe zugrunde, die Wundbehandlung, insbesondere von Wunden in der Epithelisierungsphase, weiter zu verbessern. Es soll mit der vorliegenden Erfindung ein System zur Behandlung von Wunden zur Verfügung gestellt werden, mit dem eine Behandlung möglichst wirksam durchgeführt werden kann und welche beim Patienten nicht als unangenehm empfunden wird. Ferner soll das System vorteilhaft in der Wundtherapie anwendbar sein.

Insbesondere soll die vorliegende Erfindung die Wundheilung in der Epithelisierungs- oder Regenerationsphase vorteilhaft beeinflussen, sodass beispielsweise eine kürzere Behandlungsdauer und/oder geringere Narbenbildung erreicht werden kann.

Die Aufgaben konnten unerwartet durch ein Wundsystem gemäß Anspruch 1, welches eine einen ersten Stoff umfassende erste Schicht, und einen oberhalb oder unterhalb der ersten Schicht angeordneten zweiten Stoff umfasst, gelöst werden, wobei der erste und der zweite Stoff ein unterschiedliches Standardpotential $E°$ aufweisen. Somit kann bevorzugt ein elektrischer Mikrostrom erzeugt werden.

Ein Gegenstand der Erfindung ist daher ein Wundsystem, umfassend:

(a) erste Schicht, die einen ersten Stoff umfasst;

(b) einen zweiten Stoff, der oberhalb oder unterhalb der ersten Schicht angeordnet ist

wobei der erste und der zweite Stoff ein unterschiedliches Standardpotential $E°$ aufweisen, gemessen bei 25°C; 101,3 kPa, pH=0, Ionenaktivität = 1, wobei der erste Stoff Silber und/oder ein Silbersalz ist, und wobei der zweite Stoff Zink ist, wobei die erste Schicht einen Film, einen Schaum, ein Gewebe, ein Gestrick, ein Filament, einen Vliesstoff, verwobene Materialien und/oder Kombinationen daraus umfasst, und wobei der zweite Stoff in einer zweiten Schicht enthalten ist, wobei die zweite Schicht einen Polymerschaum umfasst.

Ein nicht erfindungsgemäßer Gegenstand ist ein Stoffgemisch aus einem ersten und zweiten Stoff, wobei der erste Stoff in einer Schicht enthalten und der zweite Stoff außerhalb der ersten Schicht angeordnet ist und wobei der erste und der zweite Stoff unterschiedliche Standardpotentiale $E°$ aufweisen, wobei das Stoffgemisch zur Behandlung von Wunden während der Epithelisierungsphase eingesetzt wird.

Das neue erfindungsgemäße Wundsystem zeichnet sich durch mehrere unerwartete Vorteile aus. Durch die erfindungsgemäße Anordnung kann eine vertikale Komponente des elektrischen Feldes und ein entsprechender elektrischer Mikrostrom erzeugt werden, wodurch insbesondere während der Epithelisierungsphase eine vorteilhafte Wundheilung und/oder eine anschließende vorteilhafte Verminderung der Narbenbildung erreicht werden kann.

Die Bestandteile des erfindungsgemäßen Wundsystems werden nachstehend beschrieben.

Das erfindungsgemäße Wundsystem umfasst eine erste Schicht, welche die in Anspruch 1 beschriebenen Materialien enthält.

In einer bevorzugten Ausführungsform kann die erste Schicht ein natürliches Material enthalten, wobei ein natürliches Material bevorzugt pflanzlicher oder tierischer Herkunft ist. Beispiele für natürliche Materialien sind Polypeptide, Pektine, Seiden, Keratine, Alginate und natürliche Fasern, wie Baumwolle, Seide oder Zellstoff, sowie Gemische davon.

Alternativ bevorzugt kann die erste Schicht ein synthetisches Material enthalten. Synthetische Materialien können auch natürlichen Ursprungs sein, wobei sie durch anschließende Behandlung, wie eine chemische Umsetzung, verändert werden. Beispiele sind Polymere wie Polyamide, Polyurethane, Polyolefine, Polyvinylalkohol, Poly(meth)acrylate, Acrylate, Kunstseide, Elastomere, künstliche Cellulosen, wie Methylcellulose, Ethylcellulose und Hydroxypropylmethylcellulose, und künstliche Schäume sowie Gemische davon.

Die erste Schicht umfasst einen Film, einen Schaum, ein Gewebe, ein Gestrick, ein Filament, einen Vliesstoff (auch als "Nonwoven" bezeichnet), verwobene Materialien und/oder Kombinationen daraus.

In einer bevorzugten Ausführungsform umfasst die erste Schicht einen hydrophilen Polyurethanschaum. In der trockenen Form hat der hydrophile Polyurethanschaum einen Wasseranteil von höchstens 1 Gew.-%, bevorzugt von höchstens 0,5 Gew.-%. In feuchtem Zustand kann der hydrophile Polyurethanschaum einen Wasseranteil von mindestens 20 Gew.-% Wasser, insbesondere mindestens 30 Gew.-% Wasser und ganz besonders bevorzugt mindestens 35 Gew.-% Wasser, umfassen. Hierbei ist weiterhin bevorzugt vorgesehen, dass der Polyurethanschaum im feuchten Zustand einen Wasseranteil von höchstens 80 Gew.-% Wasser, insbesondere höchstens 70 Gew.-% und ganz besonders bevorzugt von höchstens 65 Gew.-% Wasser, umfasst.

Der Wasseranteil kann vorzugsweise in dem hydrophilen Polyurethanschaum homogen verteilt sein. Insbesondere umfasst der hydrophile Polyurethanschaum dabei einen Wasseranteil von mindestens 10 Gew.-% und höchstens 80 Gew.-% Wasser, wobei das Wasser insbesondere homogen in dem Polyurethanschaum verteilt ist.

Hierbei und im Folgenden soll im Zusammenhang der vorliegenden Erfindung - falls nichts anderes angegeben ist - jeder Gehalt eines Inhaltsstoffes in Gewichtsprozent (Gew.-%) bezogen auf das Gewicht der den Inhaltsstoff umfassenden Komponente verstanden sein.

Zur Überprüfung der Menge an Wasser einer Komponente soll im Zusammenhang mit der vorliegenden Erfindung die Norm DIN EN 14079 herangezogen werden, wobei die Menge an Wasser wie folgt berechnet wird:

$$W_w = \frac{W_g - W_t}{W_g} \bullet 100\% \qquad (1)$$

mit

$W_w$ = Gewicht des Wassers in %, bezogen auf das Gesamtgewicht der Komponente,
$W_g$ = Gewicht der Wasser enthaltenden Komponente,
$W_t$ = Gewicht der trockenen Komponente.

Damit soll im Zusammenhang der vorliegenden Erfindung unter einem Polyurethanschaum mit einem Wasseranteil von mindestens 10 Gew.-% ein solcher Polyurethanschaum verstanden sein, der mindestens 10 Gew.-% Wasser umfasst, wobei das Wasser von dem Polyurethanschaum freigesetzt werden kann. Im Unterschied hierzu ist nicht der Anteil an Wasser zu verstehen, der eventuell zur Bildung, beispielsweise bei der Polymerisation der Ausgangsprodukte des Polyurethanschaums, verwendet wird. Dieses Wasser wird kovalent gebunden und steht nicht der Wundbehandlung zur Verfügung. Darüber hinaus soll auch nicht ein Wasseranteil verstanden sein, der produktionsbedingt bei der Herstellung des Schaums verwendet wird. Dieser Wasseranteil wird nach oder während der Bildung des Polyurethanschaums dem Schaum meist durch Trocknen, beispielsweise durch Trocknen in einem Ofen, entzogen und steht somit auch nicht der Wundbehandlung zur Verfügung. Damit weist eine erfindungsgemäße Wundauflage einen Polyurethanschaum auf, der einen Wasseranteil umfasst, der deutlich einen eventuell durch die Herstellung nach Trocknung bedingten Restgehalt an Wasser übersteigt.

Weiterhin kann der hydrophile Polyurethanschaum einen Retentionswert R von mindestens 20 %, bevorzugt mindestens 30 %, insbesondere mindestens 40 % und ganz besonders bevorzugt von mindestens 50 % aufweisen.

Unabhängig hiervon kann weiterhin bevorzugt vorgesehen sein, dass der hydrophile Polyurethanschaum einen Retentionswert R von höchstens 90 %, insbesondere von höchstens 80 % und ganz besonders von höchstens 70 % aufweist. Der Retentionswert R wird dabei gemäß der folgenden Methode bestimmt.

Der Retentionswert R beschreibt die Menge Wasser, die der Polyurethanschaum in seine Polyurethanmatrix maximal einbinden kann, wobei das Wasser, das in die Poren aufgenommen werden könnte, unberücksichtigt bleibt. Der Retentionswert wird bestimmt, indem ein Probenstück von 5 cm x 5 cm (unter Normklima gelagert) aus einem hydrophilen Polyurethanschaum mit einer Dicke von höchstens 5 mm ausgestanzt und dessen Gewicht unter Normklimabedingungen vermessen wird. Das Probenstück wird hiernach einer freien Absorption mit Wasser analog DIN EN 13726-1 unterworfen. Das Wasser, das von den Poren aufgenommen wurde, wird dem Probenstück mittels einer Rolle (Gewicht 5000 g, Durchmesser 10 cm, Breite 5 cm) herausgequetscht, indem die Probe mehrmals zwischen frische Zellstofftücher gelegt und mit der Rolle überrollt wird. Dieser Vorgang wird solange wiederholt, bis keine Wasserabsorption in den Zellstofftüchern mehr erkennbar ist. Zur Bestimmung des Retentionswertes R wird der Wasseranteil $W_{ww}$, der nach dem Absorbieren und dem Ausquetschen in dem Polyurethanschaum enthalten ist, gemäß DIN EN 14079 gemessen und wie folgt berechnet:

$$R = W_{ww} = \frac{W_{gg} - W_{tt}}{W_{gg}} \bullet 100\% = 52,8\,\%$$

(gemessen nach DIN EN 14079) wobei gilt

$W_{ww}$ = das Gewicht des Wassers, das nach der Absorption und dem Ausquetschen in der Polyurethanschaum enthalten ist,

$W_{tt}$ = das Gewicht des Probenstücks nach dem Trocknen, und

$W_{gg}$ = das Gewicht des Probenstücks nach Absorption und nach Ausquetschen.

Ganz besonders bevorzugt ist ein hydrophiler Polyurethanschaum, der im feuchten Zustand einen Wasseranteil von mindestens 10 Gew.-% umfasst, wobei der Wasseranteil dem Retentionswert R des Polyurethanschaums entspricht.

Im Falle eines feuchten hydrophilen Polyurethanschaums können die Wunde sowie der in der ersten Schicht enthaltende erste Stoff befeuchtet oder mit Feuchtigkeit versorgt werden, wobei durch den Polyurethanschaum zudem eine ausreichende Absorptionskapazität vorhanden ist. Hierdurch werden die Wundheilung eventuell negativ beeinflussende Faktoren der Wundoberfläche entzogen und gleichzeitig Feuchtigkeit und Wasser in einer ausreichenden Menge zur Verfügung gestellt. Der hydrophile Polyurethanschaum ist hervorragend geeignet, um in der Epithelisierungs- oder Granulationsphase, insbesondere der Epithelisierungsphase der Wundheilung eingesetzt zu werden. Damit kann der hydrophile Polyurethanschaum in natürlicher Weise die Granulation und/oder die Epithelisierung der Wunde in besonderem Maße fördern.

In einer bevorzugten Ausführungsform kann der hydrophile Polyurethanschaum einen Wasseranteil von mindestens 10 Gew.-% und ein Quellvermögen $\Delta V_1$, von höchstens 80 % aufweisen. Insbesondere weist der hydrophile Polyurethanschaum dabei ein Quellvermögen $\Delta V_1$ von höchstens 60 %, insbesondere von höchstens 40 %, insbesondere von höchstens 30 % und ganz besonders bevorzugt von höchstens 20 % auf. Dabei kann weiterhin vorteilhaft vorgesehen sein, dass der Polyurethanschaum ein Restquellvermögen $\Delta V_1$ von mindestens 5 % aufweist. Dieses Restquellvermögen kann ausgenützt werden, damit das Wundsystem während der Absorption einen besseren Kontakt zum Wundgrund annehmen kann.

Hierbei soll unter dem Quellvermögen $\Delta V_1$ eines Polyurethanschaums die Volumenzunahme verstanden sein, die ein Polyurethanschaum, der seine Absorptionskapazität vollständig erschöpft hat, im Vergleich zu einem Polymerurethanschaum mit einem Wassergehalt von mindestens 10 Gew.-% Wasser erfährt. Das Quellvermögen soll dabei gemäß einem hierin beschriebenen Test ermittelt werden.

Das Quellvermögen $\Delta V_1$ eines Polyurethanschaums beschreibt die Volumenänderung, die ein wasserhaltiger Polyurethanschaum erfährt, nachdem er seine maximale Absorption erreicht hat. Zur Bestimmung des Quellvermögens $\Delta V_1$ werden die räumlichen Abmessungen eines Probenstücks des wasserhaltigen Polymerschaums und die räumlichen Abmessungen dieses Probenstücks nach vollständiger Absorption gemäß der freien Absorption nach DIN EN 13726-1 bestimmt. Zur Bestimmung der Dicke (Höhe) wird ein Dickenmessgerät mit 25 cm² Teller verwendet, wobei eine Belastung von 2 g/cm² gemäß EN ISO 9073-2 eingestellt wird. Die laterale Ausdehnung (Länge, Breite) wird mittels einer Schieblehre bestimmt, ohne dass das Probenstück deformiert wird. Zur Bestimmung der Ausdehnung wird das jeweilige Probenstück spannungsfrei auf eine glatte Oberfläche abgelegt. Die Volumenänderung nach Absorption entspricht dem Quellvermögen $\Delta V_1$ des wasserhaltigen Polyurethanschaums, wobei alle drei Raumrichtungen beachtet werden.

$$\Delta V_1 = \frac{V_2 - V_1}{V_1} \bullet 100\% = \frac{(l_2 \bullet b_2 \bullet h_2) - (l_1 \bullet b_1 \bullet h_1)}{(l_1 \bullet b_1 \bullet h_1)} \bullet 100\%$$

wobei gilt

$V_1$ = das Volumen des wasserhaltigen Probenstücks und
$V_2$ = das Volumen des Probenstücks nach vollständiger Absorption.

Als Polyurethanschaum kann im Zusammenhang der vorliegenden Erfindung jeder heute in der modernen Wundbehandlung übliche hydrophile Polyurethanschaum verwendet werden, der einen Wasseranteil in seine Polyurethanmatrix aufnehmen kann und eine ausreichende Absorption aufweist. Damit ist im Zusammenhang der vorliegenden Erfindung unter einem hydrophilen Polyurethanschaum ein solcher Polyurethanschaum zu verstehen, der eine Flüssigkeit in seine Polyurethanmatrix und in seine Poren aufnehmen und speichern, somit absorbieren, kann und zumindest einen Teil der aufgenommenen Flüssigkeit wieder abgeben kann. Als hydrophile Polymerschäume sind hierbei insbesondere offenporige, hydrophile Polyurethanschäume geeignet. Demgemäß umfasst eine besonders bevorzugte Wundauflage eine zweite Schicht, die einen offenporigen, hydrophilen Polyurethanschaum umfasst.

Weiterhin bevorzugt kann der hydrophile Polyurethanschaum eine durchschnittliche Porengröße von weniger als 1000 $\mu$m, insbesondere 100 $\mu$m bis 1000 $\mu$m, insbesondere 100 $\mu$m bis 500 $\mu$m und ganz besonders bevorzugt 100 $\mu$m bis 300 $\mu$m aufweisen. Hierbei kann insbesondere vorgesehen sein, dass die durchschnittliche Porengröße an der ersten Seite der zweiten Schicht gleich groß ist wie die Porengröße im Inneren der zweiten Schicht und/oder gleich groß ist wie an der zweiten Seite der zweiten Schicht. Weiterhin bevorzugte hydrophile Polyurethanschäume weisen eine Dichte von weniger als 150 kg/m$^3$, insbesondere weniger als 140 kg/m$^3$ und ganz besonders bevorzugt 50 kg/m$^3$ bis 120 kg/m$^3$ auf. Die Bestimmung der Porengröße erfolgt bevorzugt mikroskopisch, wobei ein Probenquerschnitt mikroskopiert wurde; die angegebene Porengröße entspricht dem Mittelwert von 5 zufällig ausgewählten und ausgemessenen Poren pro Probe.

Als vorteilhafte Ausführungsform hat sich gezeigt, dass im Falle, dass die erste Schicht einen hydrophilen Polyurethanschaum umfasst, eine Dicke von 0,1 bis 5,0 mm insbesondere von 0,5 bis 5,0 mm und ganz besonders bevorzugt von 0,5 bis 3,0 mm aufweist. Solche Schichtdicken zeigen einerseits die Fähigkeit auf, ein von einer Wunde abgegebenes Wundexsudat aufzunehmen und andererseits gleichzeitig eine ausreichende Menge an Wasser oder Feuchtigkeit einer Wunde bereitstellen zu können. Diese Schichtdicken können an jeder Stelle gleich sein oder in verschiedenen Bereichen verschiedene Werte annehmen. Insbesondere ist auch vorgesehen, dass die erste Schicht abgeflachte Ränder aufweist.

In einer alternativ besonders bevorzugten Ausführungsform kann die erste Schicht ein Gewebe, einen Vliesstoff, verwobene Materialien und/oder Kombinationen daraus umfassen. Besonders bevorzugt sind Gewirke (nicht erfindungsgemäß). Diese können das oben stehende natürliche oder künstliche Material enthalten.

In einer besonders bevorzugten nicht erfindungsgemäßen Ausführungsform kann die erste Schicht Polyamidgewirke enthalten. Die Polyamidgewirke können bevorzugt Polyamidfasern, insbesondere aus einem Nylon-Faden enthalten.

In einer alternativ bevorzugten Ausführungsform ist die erste Schicht wasser- beziehungsweise flüssigkeitsdurchlässig. Unter wasser- beziehungsweise flüssigkeitsdurchlässig soll verstanden werden, dass die Schicht einen Wasser- beziehungsweise Flüssigkeitsdurchgang von der einen Seite der ersten Schicht zur anderen Seite der ersten Schicht nicht vollständig unterbindet. In einer bevorzugten Ausführungsform kann die erste Schicht eine Vielzahl von Kanälen zum Durchtritt von Flüssigkeit umfassen.

In einer besonders bevorzugten Ausführungsform der vorliegenden Erfindung kann die erste Schicht einen wasser- beziehungsweise flüssigkeitsdurchlässigen Polymerschaum umfassen. Hier sind besonders Schäume geeignet, die aus Polyurethan, Polyetherurethan, Polyesterurethan, Polyether-Polyamid-Copolymeren, Polyacrylat und Polymethacrylat gefertigt werden. Sehr bevorzugt ist als Trägerschicht ein wasserdurchlässiger Polyurethanfilm oder Polyurethanschaum. Besonders bevorzugt ist als Polymerfilm ein Polyurethanfilm, Polyetherurethanfilm oder Polyesterurethanfilm. Die Polymerfilme, welche als erste Schicht verwendet werden können, weisen vorzugsweise eine Dicke von 15 bis 50 $\mu$m, mehr bevorzugt 20 bis 40 $\mu$m, insbesondere 25 bis 30 $\mu$m auf. Weiterhin kann der Polymerfilm vorzugsweise eine Wasserdampfdurchlässigkeit von 750 g/m$^2$/24h bis 5000 g/m$^2$/24h, mehr bevorzugt von 1000 g/m$^2$/24h bis 4000 g/m$^2$/24h, insbesondere von 2000 g/m$^2$/24h bis 3500 g/m$^2$/24h aufweisen.

Der erste und der zweite Stoff können in verschiedenen geometrischen Formen vorliegen. Die Grenzen dieser geometrischen Formen sind vorzugsweise klar definiert.

Die erste Schicht enthält den ersten Stoff, der Silber und/ oder ein Silbersalz ist. Der erste Stoff kann durch die den Fachmann bekannten Verfahren in die erste Schicht eingebracht werden. In einer bevorzugten Ausführungsform kann der Stoff durch Stanzen in die erste Schicht eingebracht werden. Für den bevorzugten Fall, dass die erste Schicht ein Polyamidgewirk ist, ist es bevorzugt, wenn die Polyamidfaser bzw. der Nylonfaden mit dem ersten Stoff versehen werden, bevor das Gewirk gebildet wird. Die Polyamidfaser bzw. der Nylonfaden können beispielsweise mit dem ersten Stoff ummantelt, bevorzugt teilweise ummantelt werden.

In einer weiteren bevorzugten Ausführungsform ist die erste Schicht die Wundkontaktschicht. Das heißt, dass die den ersten Stoff enthaltende Schicht auf die Wunde aufgebracht wird, vorzugsweise direkt aufgebracht wird und der außerhalb der ersten Schicht angeordnete zweite Stoff auf der wundabgewandten Seite der ersten Schicht angeordnet wird.

Der zweite Stoff ist außerhalb der ersten Schicht angeordnet. Unter außerhalb wird im Rahmen dieser Anmeldung verstanden, dass der zweite Stoff oberhalb, oder unterhalb von der ersten Schicht angeordnet ist. Durch die Anordnung kann gewährleistet werden, dass das elektrische Feld eine zur Wundoberfläche vertikale Komponente aufweist.

In einer bevorzugten Ausführungsform beträgt der elektrische Mikrostrom 1 µA bis 800 µA, bevorzugt 10 µA bis 600 µA, mehr bevorzugt 25 µA bis 400 µA, insbesondere 50 µA bis 200 µA.

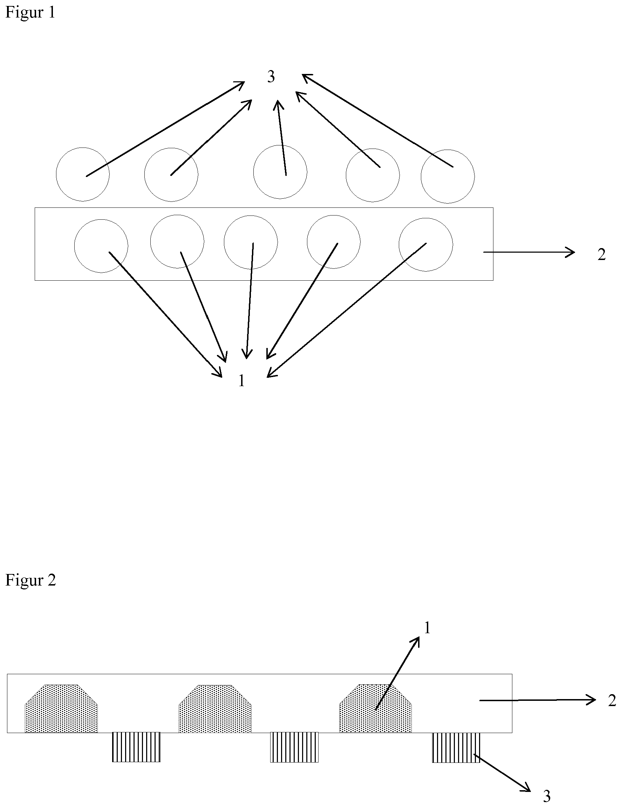

In einer bevorzugten Ausführungsform (nicht erfindungsgemäß) kann der zweite Stoff direkt auf der ersten Schicht aufgebracht werden. Hierzu kann der zweite Stoff beispielsweise in Form einer Folie, als Pulver oder Puder, bevorzugt als Puder, direkt auf die erste Schicht aufgebracht werden. In einer weiter bevorzugten Ausführungsform kann die erste Schicht vor dem Aufbringen auf die Wunde mit einer wässrigen Lösung, bevorzugt mit einer Ringerlösung, getränkt werden. Die erste Schicht ist bevorzugt die Wundkontaktschicht. Anschließend wird der zweite Stoff auf die erste Schicht aufgebracht (nicht erfindungsgemäß) sodass ein elektrischer Mikrostrom erzeugt werden kann. Eine entsprechende Ausführungsform kann durch Figur 1 (nicht erfindungsgemäß) veranschaulicht werden.

In Figur 1 bedeuten

    1 erster Stoff
    2 erste Schicht
    3 zweiter Stoff

In einer alternativ bevorzugten Ausführungsform (nicht erfindungsgemäß) kann der zweite Stoff so angeordnet sein, dass er versetzt, bevorzugt vollständig versetzt, zu dem ersten Stoff in der ersten Schicht vorliegt. Unter vollständig versetzt kann verstanden werden, dass sich, wenn der zweite Stoff senkrecht zur Schichtoberfläche in die erste Schicht überführt würde, die geometrischen Formen der ersten und zweiten Stoffe nicht überschneiden würden. Eine entsprechende Ausführungsform kann durch Figur 2 (nicht erfindungsgemäß) veranschaulicht werden.

In Figur 2 bedeuten

    1 erster Stoff
    2 erste Schicht
    3 zweiter Stoff

In einer alternativ bevorzugten Ausführungsform (nicht erfindungsgemäß) kann auf der ersten Schicht eine Trennschicht aufgebracht werden und auf diese Trennschicht kann der zweite Stoff beispielsweise in Form einer Folie, als Pulver oder Puder, bevorzugt als Puder, aufgebracht werden. Die Trennschicht verhindert, dass der zweite Stoff, vorzugsweise der zweite Stoff in ungelöstem Zustand, insbesondere wenn er in Puderform vorliegt, in die erste Schicht eindringen kann. Weiterhin ist bevorzugt, dass die Trennschicht eine flüssigkeitsdurchlässige, insbesondere eine wasserdurchlässige, Schicht ist. Unter flüssigkeits- bzw. wasserdurchlässige soll das Gleiche wie obenstehend beschrieben verstanden werden.

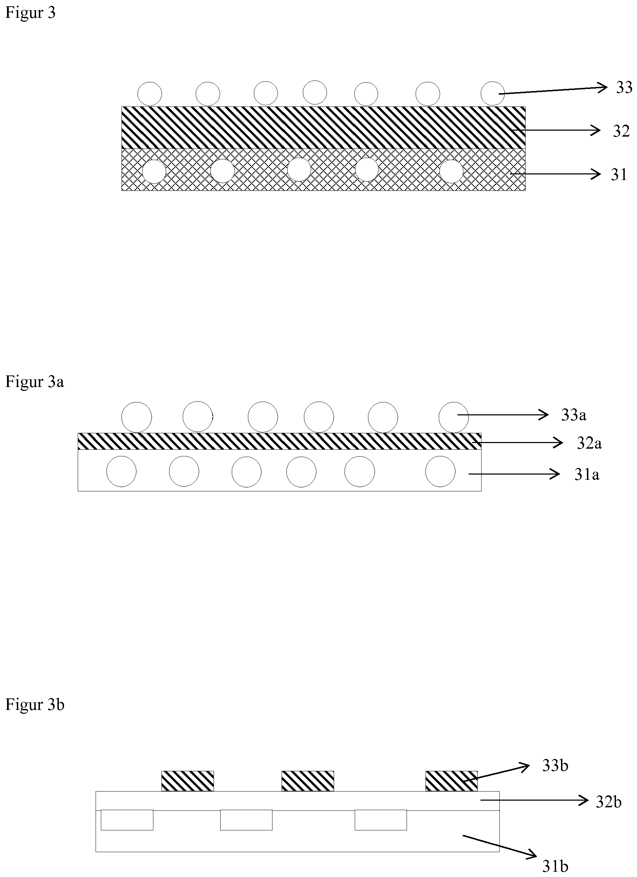

In einer besonders bevorzugten Ausführungsform (nicht erfindungsgemäß) kann die Trennschicht einen wasser- beziehungsweise flüssigkeitsdurchlässigen Film umfassen. Sehr bevorzugt umfasst die Trennschicht ein wasserdurchlässiges Polyurethan, bevorzugt einen Polyurethanfilm. Besonders bevorzugt sind ein Polyetherurethanfilm oder Polyesterurethanfilm. Die Trennschicht kann eine Wasserdampfdurchlässigkeit von $750 \text{ g/m}^2/24\text{h}$ bis $5000 \text{ g/m}^2/24\text{h}$, mehr bevorzugt von $1000 \text{ g/m}^2/24\text{h}$ bis $4000 \text{ g/m}^2/24\text{h}$, insbesondere von $2000 \text{ g/m}^2/24\text{h}$ bis $3500 \text{ g/m}^2/24\text{h}$ aufweisen. Eine entsprechende Ausführungsform kann durch Figur 3 (nicht erfindungsgemäß) veranschaulicht werden.

In Figur 3 bedeuten

    31    eine einen ersten Stoff enthaltende erste Schicht
    32    Trennschicht
    33    zweiter Stoff

In einer alternativen Ausführungsform (nicht erfindungsgemäß) kann die Trennschicht auch ein wie oben beschriebener hydrophiler Polyurethanschaumstoff in getrocknetem Zustand sein. Der hydrophile Polyurethanschaumstoff kann vorzugsweise kurz vor der Anwendung mit wässriger Lösung, bevorzugt Ringerlösung, getränkt werden. Eine entsprechende Ausführungsform kann durch Figur 3a (nicht erfindungsgemäß) veranschaulicht werden.

In Figur 3a bedeuten

31a  eine einen ersten Stoff enthaltende erste Schicht
32a  Trennschicht, welche vor der Anwendung in Ringerlösung getränkt werden kann
33a  zweiter Stoff

In einer weitern alternativ bevorzugten Ausführungsform (nicht erfindungsgemäß) kann der zweite Stoff so angeordnet sein, dass er versetzt, bevorzugt vollständig versetzt, zu dem ersten Stoff in der ersten Schicht vorliegt Eine entsprechende Ausführungsform kann durch Figur 3b (nicht erfindungsgemäß) veranschaulicht werden.

In Figur 3b bedeuten

31b  eine einen ersten Stoff enthaltende erste Schicht
32b  Trennschicht
33b  zweiter Stoff

In einer weiteren bevorzugten Ausführungsform wird auf der ersten Schicht eine zweite Schicht angeordnet, wobei die zweite Schicht den zweiten Stoff enthält. Die erste und zweite Schicht können bevorzugt durch ein Adhäsiv verbunden werden. Alternativ oder zusätzlich kann zwischen der ersten und zweiten Schicht noch eine wie oben stehend beschriebene Trennschicht angebracht sein.

Die zweite Schicht umfasst einen Polymerschaum. Weiterhin kann die zweite Schicht die gleichen Merkmale/Eigenschaften wie die erste Schicht aufweisen.

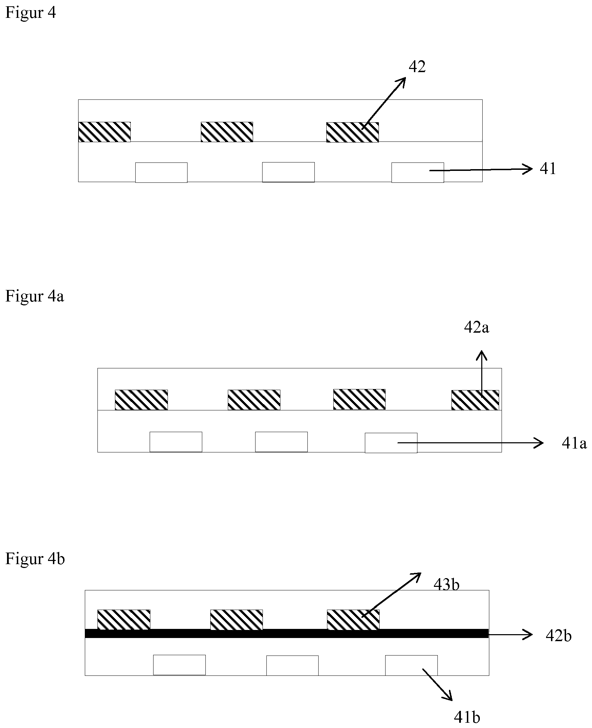

In einer bevorzugten Ausführungsform kann auf der ersten Schicht eine zweite Schicht angeordnet sein, wobei die zweite Schicht den zweiten Stoff enthält und wobei der zweite Stoff in der zweiten Schicht so angeordnet ist, dass er vollständig versetzt zu dem ersten Stoff in der ersten Schicht vorliegt. Die beiden Schichten können bevorzugt durch ein Adhäsiv miteinander verbunden sein, sodass sie nicht gegeneinander verschiebbar sind. Eine entsprechende Ausführungsform kann durch Figur 4 veranschaulicht werden.

In Figur 4 bedeuten

41  erster Stoff, der in einer ersten Schicht enthalten ist
42  zweiter Stoff, der in einer zweiten Schicht enthalten ist

In einer alternativ bevorzugten Ausführungsform kann auf der ersten Schicht eine zweite Schicht angeordnet sein, wobei die zweite Schicht den zweiten Stoff enthält. Die beiden Schichten können bevorzugt durch ein Adhäsiv miteinander verbunden sein, sodass sie nicht gegeneinander verschiebbar sind. Weiterhin kann bevorzugt der zweite Stoff in der zweiten Schicht so angeordnet ist, dass er teilweise versetzt zu dem ersten Stoff in der ersten Schicht vorliegt. Eine entsprechende Ausführungsform kann durch Figur 4a veranschaulicht werden.

In Figur 4a bedeuten

41a  erster Stoff, der in einer ersten Schicht enthalten ist
42a  zweiter Stoff, der in einer zweiten Schicht enthalten ist

In einer alternativ bevorzugten Ausführungsform kann auf der ersten Schicht eine zweite Schicht angeordnet sein, wobei die zweite Schicht den zweiten Stoff enthält. Die erste und zweite Schicht sind durch eine Adhäsivschicht, welche vorzugsweise flüssigkeitsdurchlässig ist, verbunden. Weiterhin kann bevorzugt der zweite Stoff in der zweiten Schicht so angeordnet ist, dass er vollständig versetzt zu dem ersten Stoff in der ersten Schicht vorliegt. Eine entsprechende Ausführungsform kann durch Figur 4b veranschaulicht werden.

In Figur 4b bedeuten

41b  erster Stoff, der in einer ersten Schicht enthalten ist
42b  Adhäsivschicht
43b  zweiter Stoff, der in einer zweiten Schicht enthalten ist

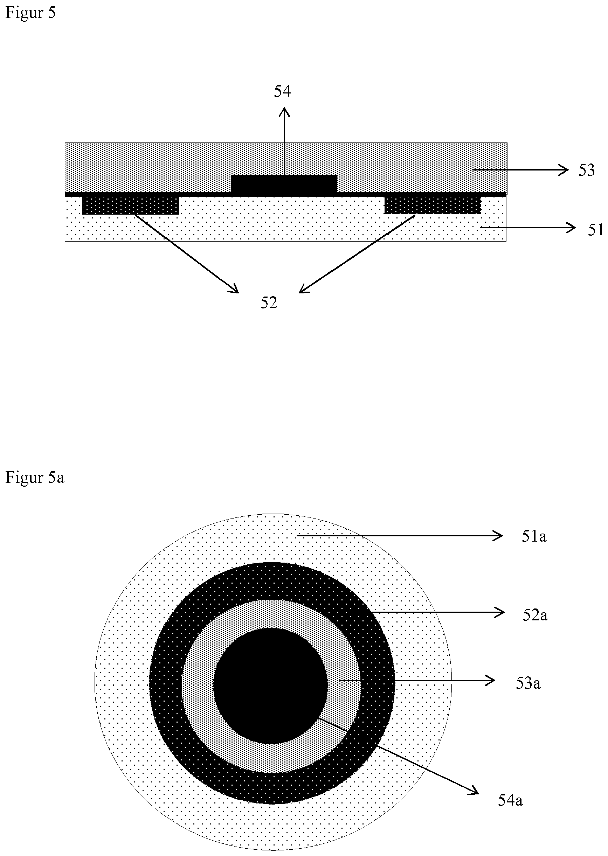

In einer alternativ bevorzugten Ausführungsform kann auf der ersten Schicht eine zweite Schicht angeordnet sein, wobei die zweite Schicht den zweiten Stoff enthält. Zusätzlich kann zwischen der ersten und der zweiten Schicht eine Trennschicht angebracht werden. Bevorzugt sind sowohl die erste als auch die zweite Schicht eine wie oben beschriebene Schaumstoffschicht, bevorzugt hydrophile Polyurethanschaumstoffschicht. Weiterhin kann der zweite Stoff in der zweiten Schicht bevorzugt zentral angeordnet sein, sodass er als eine Zentralelektrode angesehen werden kann. Weiterhin kann der erste Stoff in der ersten Schicht bevorzugt ringförmig angeordnet sein, sodass er als eine Ringelektrode angesehen werden kann. Es ist bevorzugt, dass der Durchmesser des Ringes der Ringelektrode größer ist als der Durchmesser der Zentralelektrode. Eine entsprechende Ausführungsform kann durch die Figuren 5 und 5a veranschaulicht werden, wobei Figur 5a eine Draufsicht ist.

In Figur 5 bedeuten

51  zweite Schicht
52  Ringelektrode
53  erste Schicht
54  Zentralelektrode

Figur 5a bedeuten

51a  zweite Schicht
52a  Ringelektrode
53a  erste Schicht
54a  Zentralelektrode

In einer alternativ bevorzugten Ausführungsform kann auf der ersten Schicht eine zweite Schicht angeordnet sein, wobei die zweite Schicht den zweiten Stoff enthält. Die erste Schicht ist bevorzugt eine Gewebeschicht in der der erste Stoff angebracht ist. Bevorzugt ist die zweite Schicht eine Schaumstoff-Hydrogel-Schicht. Der Schaumstoffteil der Schaumstoff-Hydrogel-Schicht ist bevorzugt ein hydrophiler Polyurethanschaumstoff wie oben beschrieben. Weiterhin umfasst die Schaumstoff-Hydrogel-Schicht bevorzugt eine Hydrokolloidmatrix. Diese Hydrokolloidmatrix kann aus einer klebenden Polymermatrix bestehen, in die Hydrokolloidpartikel dispergiert sind. Gemäß der vorliegenden Erfindung soll unter einem Hydrokolloid ein Material verstanden sein, das ein hydrophiles synthetisches oder natürliches Polymermaterial ist, das löslich oder absorbierend und/oder quellend in Wasser ist.

Vorzugsweise umfasst das Hydrokolloid ein synthetisches oder natürliches Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate, wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargum, Gelatine oder Mischungen hiervon. Das Hydrokolloid kann sowohl in Form von Fasern als auch in Form von Partikeln oder Fasern innerhalb einer Matrix vorliegen. Insbesondere kann das Hydrokolloid in Form von Partikeln in einer Polymermatrix, vorzugsweise in Form einer klebenden Polymermatrix, vorliegen. Die Polymermatrix umfasst dabei mindestens ein Co-Blockpolymer, ausgewählt aus der Gruppe der AB-Diblock-Copolymere und/oder ABA-Triblock-Copolymere, das aus den Monomeren Styrol, Butadien und Isopren aufgebaut ist.

Gemäß einer weiteren Ausführungsform der vorliegenden Erfindung kann weiterhin vorgesehen sein, dass die Schaumstoff-Hydrogel-Schicht eine wasserhaltige Hydrogelmatrix umfasst. Diese Hydrogelmatrix kann vorzugsweise mindestens 20 Gew.-%, insbesondere mindestens 30 Gew.-%, insbesondere mindestens 40 Gew.-% und ganz besonders bevorzugt mindestens 50 Gew.-% Wasser umfassen, wobei die Hydrogelmatrix weiterhin bevorzugt höchstens 90 Gew.-% und weiterhin bevorzugt höchsten 80 Gew.-% Wasser umfasst. Als wasserhaltige Hydrogelmatrices können im Zusammenhang der vorliegenden Erfindung insbesondere Hydrogelmatrices verwendet werden, die eine zusammenhängende, diskrete Schicht bilden und unter Druck kein Wasser abgeben. Insbesondere sind im Zusammenhang der vorliegenden Erfindung Hydrogelmatrices geeignet, die ein Polyurethan-Polyharnstoff-Copolymer umfassen. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelmatrices sind gut geeignet Wasser einzulagern und dieses Wasser an eine Wunde abzugeben. Weiterhin ist bevorzugt, dass die wasserhaltige Hydrogelmatrix weiterhin mindestens einen mehrwertigen Alkohol aus der Gruppe der zweiwertigen, dreiwertigen, vierwertigen, fünfwertigen oder sechswertigen Alkohole umfassen kann. Insbesondere kann der Alkohol gewählt werden aus der Gruppe der Glykole, insbesondere Ethylenglykol oder Propylenglykol, sowie Sorbitol oder Glycerin oder Mischungen hiervon. Diese Alkohole sind hervorragend als Feuchtigkeitsspender geeignet und stellen somit für die die Wunde umgebende Haut eine pflegende Komponente dar. Dabei kann die wasserhaltige Hydrogelmatrix insbesondere 0 bis 50 Gew.-% eines mehrwertigen Alkohols umfassen. Insbesondere umfasst die Hydrogelmatrix 5 bis 40 Gew.-% eines mehrwertigen Alkohols und ganz besonders bevorzugt 10 bis 30 Gew.-% eines mehrwertigen Alkohols.

Darüber hinaus kann vorgesehen sein, dass die wasserhaltige Hydrogelmatrix insbesondere mindestens ein Salz umfasst. Insbesondere ist hierbei vorgesehen, dass die Hydrogelmatrix ein anorganisches Salz umfasst. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle. Ganz besonders bevorzugt umfasst die Hydrogelmatrix Natriumchlorid, Kaliumchlorid, Magnesiumchlorid, Kalziumchlorid oder Mischungen hiervon. Diese Salze simulieren in besonders guter Weise das Elektrolyt-Gemisch in einem von einer Wunde abgegebenen Wundserum. Damit stellt eine diese Salze umfassende Hydrogelmatrix einer Wunde ein besonders wundheilungsförderndes Klima zur Verfügung.

Hierbei kann vorgesehen sein, dass die Hydrogelmatrix 0 bis 5 Gew.-% mindestens eines Salzes umfasst. Insbesondere umfasst die Hydrogelmatrix 0,1 bis 3 Gew.-% eines Salzes und ganz besonders bevorzugt 0,5 bis 1,5 Gew.-% eines Salzes.

Insgesamt kann gemäß der vorliegenden Erfindung vorgesehen sein, dass die wasserhaltige Hydrogelmatrix bevorzugt mindestens 20 Gew.-% Wasser und mindestens 10 Gew.-% Polyurethan-Polyharnstoff-Copolymer umfasst. Eine weiterhin bevorzugte Hydrogelmatrix umfasst mindestens 20 Gew.-% Wasser und mindestens 15 Gew.-% Polyurethan-Polyharnstoff-Copolymer. Hierbei kann weiterhin bevorzugt vorgesehen sein, dass die Hydrogelmatrix aus 6 bis 60 Gew.-% eines Präpolymers mit aliphatischen Diisocyanat-Gruppen, 4 bis 40 Gew.-% Polyamin auf Polyethylenoxidbasis, 0 bis 50 Gew.-% eines mehrwertigen Alkohols, 0 bis 5 Gew.-% mindestens eines Salzes ausgewählt aus der Gruppe Natriumchlorid, Kaliumchlorid, Magnesiumchlorid, Kalziumchlorid oder Mischungen hiervon und mindestens 20 Gew.-% Wasser gebildet wird.

Weiterhin bevorzugt kann vorgesehen sein, dass die Hydrogelmatrix aus 6 bis 30 Gew.-% eines Präpolymers mit aliphatischen Diisocyanat-Gruppen, 4 bis 20 Gew.-% Diamin auf Polyethylenoxidbasis, 10 bis 30 Gew.-% eines mehrwertigen Alkohols, ausgewählt aus der Gruppe bestehend aus Propylenglycol und/oder Glycerin, 0,5 bis 1,5 Gew.-% eines Salzes, ausgewählt aus der Gruppe Natriumchlorid, Kaliumchlorid, Magnesiumchlorid, Kalziumchlorid oder Mischungen hiervon, und mindestens 30 Gew.-% Wasser gebildet wird.

Ganz besonders bevorzugt kann vorgesehen sein, dass die Hydrogelmatrix aus 6 bis 20 Gew.-% Präpolymer mit Isophorondiisocyanat-Enden, 4 bis 15 Gew.-% Diamin auf Polyethylenoxidbasis, 15 bis 20 Gew.-% Polypropylenglycol und/oder Glycerin, 0,5 bis 1,5 Gew.-% eines Salzes, ausgewählt aus der Gruppe Natriumchlorid, Kaliumchlorid, Magnesiumchlorid, Kalziumchlorid oder Mischungen hiervon, und mindestens 40 Gew.-% Wasser gebildet wird. Diese Hydrogelmatrix weist eine freie Absorption $A_3$ (gemessen nach DIN EN 13723-1 (2002)) von mindestens 1 g/g und höchstens 5 g/g auf, stellt ein nicht reizendes, flüssigkeitsabsorbierendes, vor Bakterien schützendes, polsterndes, hautartiges Medium bereit und ist somit besonders gut als Wundkontaktschicht geeignet. Weiterhin bevorzugt kann die Hydrogelmatrix Kanäle umfassen, insbesondere konische Kanäle, zum Durchtritt von Flüssigkeiten von der ersten zur zweiten Seite. Hierdurch kann insbesondere ein verbesserter Durchtritt für Wundexsudat bereitgestellt werden. Hierbei ist besonders bevorzugt vorgesehen, dass die Kanäle einen elliptischen oder einen kreisförmigen Querschnitt aufweisen, d.h. dass die Kanäle eine kreisförmige oder elliptische Öffnung sowohl an der der ersten als auch an der zweiten Seite der Hydrogelmatrix aufweisen, wobei die kreisförmige oder elliptische Öffnung auf der ersten und der zweiten Seite verschieden groß sind. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Dabei ist ganz besonders bevorzugt vorgesehen, dass die erste Seite Öffnungen aufweist, die im Vergleich zu den auf der zweiten Seite befindlichen Öffnungen größer sind. Gemäß einer Weiterbildung der Erfindung kann auch vorgesehen sein, dass die Hydrogelmatrix Öffnungen aufweist, die einen Durchmesser von 0,5 bis 5 mm aufweisen. Insbesondere weist die Hydrogelmatrix Öffnungen auf, die einen Durchmesser von 1 bis 3 mm aufweisen. Ganz besonders bevorzugt weist die Hydrogelmatrix auf der wundzugewandten ersten Seite Öffnungen auf, die einen Durchmesser von 1 bis 3 mm aufweisen, wobei die zweite Seite der Wundkontaktschicht oder der Hydrogelmatrix in direktem Kontakt mit dem Polyurethanschaum steht. Der zweite Stoff kann bevorzugt in Form einer das entsprechende Ion des zweiten Stoffes enthaltenden Lösung in der zweiten Schicht vorhanden sein. Eine entsprechende Ausführungsform kann durch die Figur veranschaulicht werden.

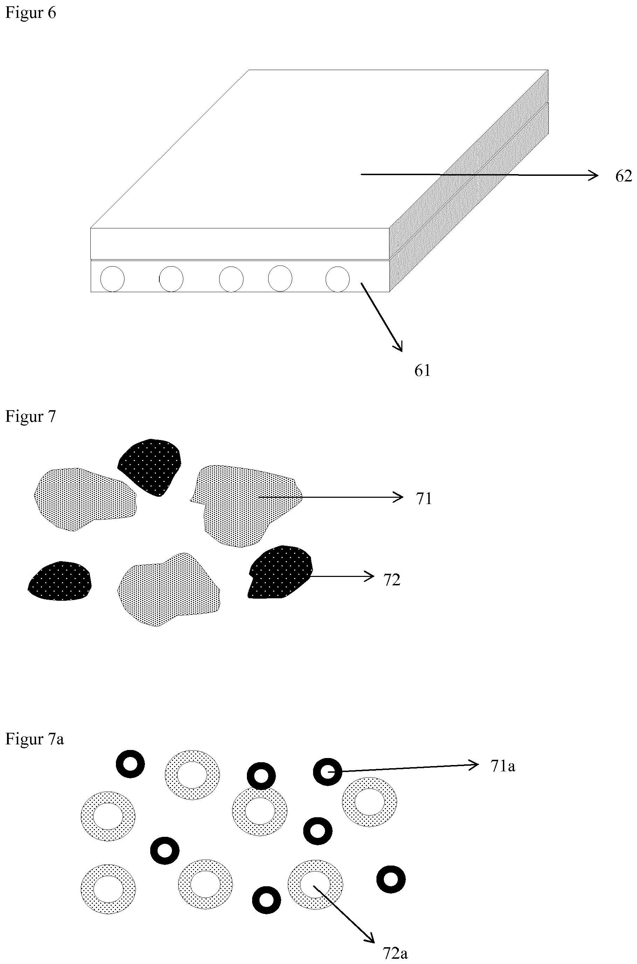

In Figur 6 (nicht erfindungsgemäß) bedeuten

61 eine einen ersten Stoff enthaltende erste Schicht
62 eine zweite Schicht, die vor der Anwendung mit einer Lösung, welche ein oder mehrere Salze des zweiten Stoffes enthält, getränkt wird.

In einer bevorzugten Ausführungsform kann die erste, den ersten Stoff enthaltenden Schicht in Form von Partikeln vorliegen. Der zweite Stoff kann vorzugsweise in einer zweiten Schicht enthalten sein, welche bevorzugt ebenfalls in Form von Partikeln vorliegen kann. Die erste und die zweite Schicht können vorzugsweise ein wie vorstehend beschriebenes Hydrogel oder eine hydrophile Matrix, vorzugsweise einen Schaum, enthalten.

In einer mehr bevorzugten Ausführungsform kann der erste Stoff in Partikeln, welche eine Hydrogelmatrix aus Poly(meth)acrylsäure und/oder Poly(meth)acrylaten umfassen, enthalten sein (nicht erfindungsgemäß), wobei diese Partikel vorzugsweise einen Durchmesser von 3 bis 4 mm aufweisen können. Der zweite Stoff kann bevorzugt in Partikeln, welche eine Hydrogelmatrix aus Carboxymethylcellulose umfassen, enthalten sein (nicht erfindungsgemäß), wobei diese Partikel vorzugsweise einen Durchmesser von 1 bis 2,5 mm aufweisen können. Bei der Anwendung können sowohl die den ersten Stoff enthaltenden ersten Partikel als auch die den zweiten Stoff enthaltenden zweiten Partikel direkt auf/in die Wunde appliziert werden, wodurch eine hohe Applikationsflexibilität gewährleistet wird (nicht erfindungsgemäß). Eine entsprechende Ausführungsform kann durch die Figur 7 (nicht erfindungsgemäß) veranschaulicht werden.

In Figur 7 bedeuten

71    eine einen ersten Stoff enthaltende erste Schicht in Partikelform
72    eine einen zweiten Stoff enthaltende zweite Schicht in Partikelform

In einer alternativ mehr bevorzugten Ausführungsform kann der erste Stoff in Partikeln, die einen hydrophilen Schaum umfassen, enthalten sein (nicht erfindungsgemäß). Weiterhin kann der zweite Stoff ebenfalls in Partikeln, die einen hydrophilen Schaum umfassen, enthalten sein (nicht erfindungsgemäß), wobei vorzugsweise kein Partikel sowohl den ersten als auch den zweiten Stoff enthält. Wie vorstehend beschrieben können bei der Anwendung sowohl die den ersten Stoff enthaltenden ersten Partikel als auch die den zweiten Stoff enthaltenden zweiten Partikel direkt auf/in die Wunde appliziert werden (nicht erfindungsgemäß). Eine entsprechende Ausführungsform kann durch die Figur 7a (nicht erfindungsgemäß) veranschaulicht werden.

In Figur 7a bedeuten

71a    eine einen ersten Stoff enthaltende erste Schicht in Partikelform
72a    eine einen zweiten Stoff enthaltende zweite Schicht in Partikelform

Der erste Stoff ist Silber und/oder ein Silbersalz. Beispiele für geeignete Silbersalze sind Silberhalogenide, wie Silberfluorid, Silberchlorid, Silberbromid, Silberiodid, Silberoxid, und Gemische davon. Es ist besonders bevorzugt, dass der erste Stoff eine Kombination aus Silber und dem entsprechenden Silbersalz ist. Silber ist ein Metall, welches ein Standardpotential $E°$ von 0,80 Volt aufweist. Für gewöhnlich werden Silberkationen als Oxidationsmittel eingesetzt, wobei sie unter Elektronenaufnahme zu Silber reduziert werden. Silber wirkt bakterizid und kann aus diesem Grund vorteilhaft im Rahmen einer Wundbehandlung/Wundheilung eingesetzt werden. Silberionen können ebenfalls in der Wundtherapie eingesetzt werden und wirken zudem desinfizierend. Weiterhin können Silber und Silberionen durch Hemmung des Bakterienwachstums unangenehme Gerüche verhindern.

Der zweite Stoff ist Zink. Zink ist ein Metall, welches ein Standardpotential $E°$ von -0,76 Volt aufweist. Zink wird gewöhnlich als Reduktionsmittel eingesetzt, wobei Zink unter Elektronenabgabe zu $Zn^{2+}$ oxidiert wird. Zink zählt zu den essentiellen Spurenelementen für den menschlichen und tierischen Körper.

In einer bevorzugten Ausführungsform kann das Wundsystem in der Epithelisierungsphase der Wundheilung eingesetzt werden. Es hat sich heraus gestellt, dass die Anwendung eines geringen elektrischen Stroms eine vorteilhafte Auswirkung auf den Heilungsprozess hat. So kann dieser bevorzugt verkürzt werden und/oder die Narbenbildung kann vorteilhaft reduziert werden.

Ein weiterer Gegenstand der Erfindung ist ein erster und zweiter Stoff zur Verwendung in der therapeutischen Behandlung von Wunden am menschlichen und tierischen Körper, wobei der erste Stoff in einer Schicht enthalten ist und wobei der zweite Stoff oberhalb oder unterhalb der ersten Schicht angeordnet ist und wobei der erste und der zweite Stoff ein unterschiedliches Standardpotential $E°$ aufweisen, gemessen bei 25°C; 101,3 kPa, pH=0; Ionenaktivität = 1, wobei der erste Stoff Silber und/ oder ein Silbersalz ist, und wobei der zweite Stoff Zink ist, wobei die erste Schicht einen Film, einen Schaum, ein Gewebe, ein Gestrick, ein Filament, einen Vliesstoff, verwobene Materialien und/oder Kombinationen daraus umfasst, und wobei der zweite Stoff in einer zweiten Schicht enthalten ist, wobei die zweite Schicht einen Polymerschaum umfasst. Der in der ersten Schicht enthaltene erste Stoff und der oberhalb oder unterhalb der ersten Schicht angeordnete zweite Stoff können somit ein Stoffgemisch darstellen, welches durch chemische und physikalische Interaktion mit menschlichen Körperzellen die Wundheilung positiv beeinflusst.

Erster und zweiter Stoff sowie die erste Schicht weisen bevorzugt die oben beschriebenen Eigenschaften auf.

In einer bevorzugten Ausführungsform werden der erste in der ersten Schicht enthaltene Stoff und der zweite oberhalb oder unterhalb der ersten Schicht angeordnete Stoff zur Verwendung zur Behandlung von Wunden in der Epithelisierungsphase eingesetzt (nicht erfindungsgemäß). Hierbei ergeben sich unerwartet die vorstehend genannten vorteilhaften Effekte bei der Wundheilung.

Wie einleitend beschrieben kann der Heilungsprozess unabhängig von der Art der Wunde in unterschiedliche Phasen typisiert werden. Im Fachgebiet wird zwischen Reinigungs- (Entzündungsphase, Inflammation), Granulations- (Proliferation) und Epithelisierungsphase unterschieden. Gemeinsames Merkmal ist, dass unterschiedliche Zelltypen miteinander interagieren, aktiviert werden und proliferieren.

In der Reinigungsphase wird die Blutung üblicherweise durch die Gerinnungskaskade gestoppt. Es bilden sich üblicherweise Gerinnsel aus Fibrinmolekülen, Fibronektin, Vitronectin und Thrombospondin. Sie können als Leitstruktur für einwandernde Zellen dienen. Gleichzeitig können die Thrombozyten im Gerinnsel Wachstumsfaktoren und Zytokine ausschütten und locken damit immunkompetente Zellen an den Ort der Gewebeverletzung. Es kommt gegebenenfalls zu einer lokalen Entzündung, bei der neutrophile Granulozyten eingedrungene Erreger abwehren. Außerdem wird gegebenenfalls abgestorbenes Zellmaterial durch Proteasen beseitigt. In dieser Phase werden meist große Exsudatmengen abgegeben, die die Selbstreinigung der Wunde unterstützen.

Die Granulationsphase ist dadurch gekennzeichnet, dass Monozyten, Endothelzellen und/oder Fibroblasten entlang der provisorischen Fibrinmatrix in das Wundareal einwandern. An den Rändern des frühen Granulationsgewebes können Zellen nekrotisches Gewebe abbauen und am Übergang zum Normalgewebe mit der Neusynthese einer extrazellulären Matrix beginnen. Nach einigen Tagen nimmt die Zellzahl in dieser Matrix massiv zu. Bindegewebe und Blutgefäße werden normalerweise sichtbar. Gegebenenfalls wird neues Kollagen synthetisiert. Während der zweiten Phase werden - sofern erforderlich - neue Gefäße gebildet.

Die Epithelisierungsphase ist, wie vorstehend erwähnt, die dritte Phase der Wundheilung. Sie ist bevorzugt dadurch charakterisiert, dass Epithelzellen in der Wunde erkennbar sind. Bevorzugt überziehen Epithelzellen das noch unfertige Bindegewebe.

Bevorzugt bildet sich, ausgehend vom intakten Epithelgewebe an den Wundrändern, die neue Epidermis. Bevorzugt sorgen ferner Myofibroblasten für eine Kontraktion der Wundränder. Keratinozyten - z.B. vom Wundrand und/oder den Haarwurzelscheiden - können ein neues Epithel bilden.

Bevorzugt wird die Granulationsphase dadurch charakterisiert, dass das Granulationsgewebe wasser- und gefäßärmer wird. Die Kollagenfasern können ausreifen und sich zu Narbengewebe umbilden (Maturation). Es kann dabei gleichzeitig eine Wundkontraktion erfolgen. Bevorzugt haben Fibroblasten ihre Aufbauarbeit beendet und wandeln sich in Fibrozyten und Myofibroblasten um. Die Epithelisierung geht besonders bevorzugt vom Wundrand aus. Hierbei überhäuten bevorzugt die sich neu bildenden Keratinozyten das Granulationsgewebe mit einem zunächst noch sehr feinen Epithel.

Bevorzugt erfolgt die Epithelisierung in einer glatten, feuchten und gut durchbluteten Kriechfläche. Unter Kriechfläche versteht man in diesem Zusammenhang ein ausgebildetes und sauberes Granulationsgewebe, das auf Wundrandniveau reicht. Es sollte im Wesentlichen frei von Nekrosen, Senken, Wundhöhlen oder Hypergranulationen sein. Für die Behandlung einer derartigen Kriechfläche ist das erfindungsgemäße Wundsystem besonders vorteilhaft.

In einer bevorzugten Ausführungsform dauert die Epithelisierungsphase drei Tage bis drei Wochen, bevorzugt fünf Tage bis drei Wochen.

In einer bevorzugten Ausführungsform wird bei der Verwendung des in einer ersten Schicht enthaltenen Stoffes und des außerhalb der ersten Schicht angeordneten zweiten Stoffes die Wunde für drei bis fünf Tage befeuchtet (nicht erfindungsgemäß).

Anschließend kann ein Wechsel des Wundsystems erfolgen.

Weiterhin stellt die Erfindung ein Kit als Wundsystem bereit, umfassend ein erfindungsgemäßes Wundsystem, wobei die den ersten Stoff enthaltende erste Schicht und der zweite Stoff getrennt und erst direkt vor der Anwendung zum Wundsystem kombiniert werden (nicht erfindungsgemäß).

Vorzugsweise liegt das abgepackte erfindungsgemäße Wundsystem in steriler Form vor. Die Sterilisation kann durch eine Gegendruckdampfsterilisation oder durch andere, dem Fachmann als geeignet bekannte Sterilisationsmethoden erfolgen.

Zudem kann das Kit bevorzugt eine wässrige Lösung zum Aktivieren des Wundsystems umfassen (nicht erfindungsgemäß). Bei der wässrigen Lösung handelt es sich bevorzugt um eine wie oben beschriebene salzhaltige, wässrige Lösung. Insbesondere kann es sich um eine Ringerlösung handeln. In diesem Zustand kann das auf die Wunde aufgebrachte Wundsystem die Wunde feuchthalten und zudem kann ein elektrischer Mikrostrom weiter zur vorteilhaften Wundheilung beitragen.

Das Kit (nicht erfindungsgemäß) kann weitere optionale Bestandteile, wie beispielsweise eine oder mehrere zusätzliche Druckverteilungsschichten, Klebemittel zum Fixieren des Verbands, Abdichtmittel zur Herstellung einer luftundurchlässigen Abdichtung des Verbandes, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung, enthalten.

Ein weiterer Aspekt der vorliegenden Anmeldung ist ein Wundsystem, welches eine Trennschicht enthält, wobei der erste und der zweite Stoff auf gegenüberliegenden Seiten angebracht sind (nicht erfindungsgemäß).

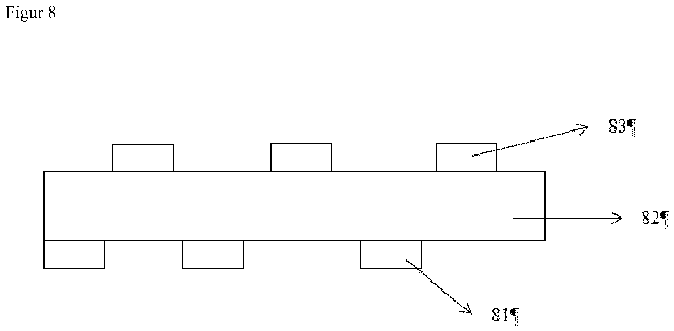

Die Trennschicht kann eine beliebige der vorstehend erwähnten Schichten sein, wobei sie bevorzugt wasser- bzw. flüssigkeitsdurchlässig ist. In einer bevorzugten Form werden der erste und der zweite Stoff jeweils direkt auf die Trennschicht aufgebracht. Es ist bevorzugt, dass der erste und der zweite Stoff versetzt, vorzugsweise vollständig versetzt, voneinander angeordnet sind. Bei einer vollständig versetzten Anordnung kann die Schicht Poren aufweisen, bei denen nur auf einer Seite ein Stoff angeordnet ist, was die Möglichkeit einer spontanen Entladung deutlich verringert. Eine entsprechende Ausführungsform kann durch die Figur 8 (nicht erfindungsgemäß) veranschaulicht werden.

In Figur 8 bedeuten

81   erster Stoff
82   Trennschicht
83   zweiter Stoff

**Patentansprüche**

1.  Wundsystem umfassend:

    (a) erste Schicht, die einen ersten Stoff enthält,
    (b) einen zweiten Stoff, der oberhalb oder unterhalb der ersten Schicht angeordnet ist,

    wobei der erste und der zweite Stoff ein unterschiedliches Standardpotential $E°$ aufweisen, gemessen bei 25°C; 101,3 kPa, pH=0, Ionenaktivität = 1, wobei der erste Stoff Silber und/oder ein Silbersalz ist, und wobei der zweite Stoff Zink ist, wobei die erste Schicht einen Film, einen Schaum, ein Gewebe, ein Gestrick, ein Filament, einen Vliesstoff, verwobene Materialien und/oder Kombinationen daraus umfasst, und
    wobei der zweite Stoff in einer zweiten Schicht enthalten ist, wobei die zweite Schicht einen Polymerschaum umfasst.

2.  Wundsystem nach Anspruch 1, wobei die erste Schicht einen Polymerschaum umfasst.

3.  Wundsystem nach Anspruch 1 oder 2, wobei die erste Schicht ein Polyamidgewirk umfasst.

4.  Wundsystem nach Anspruch 1, wobei die erste und die zweite Schicht den gleichen Polymerschaum umfassen.

5.  Erster und zweiter Stoff zur Verwendung in der therapeutischen Behandlung von Wunden am menschlichen oder tierischen Körper, wobei der erste Stoff in einer ersten Schicht enthalten und der zweite Stoff oberhalb oder unterhalb der ersten Schicht angeordnet ist, sowie ein unterschiedliches Standardpotential $E°$ aufweisen, gemessen bei 25°C; 101,3 kPa pH=0, Ionenaktivität =1, wobei der erste Stoff Silber und/oder ein Silbersalz ist, und wobei der zweite Stoff Zink ist,
    wobei die erste Schicht einen Film, einen Schaum, ein Gewebe, ein Gestrick, ein Filament, einen Vliesstoff, verwobene Materialien und/oder Kombinationen daraus umfasst, und
    wobei der zweite Stoff in einer zweiten Schicht enthalten ist, wobei die zweite Schicht einen Polymerschaum umfasst.

6.  Erster und zweiter Stoff zur Verwendung nach Anspruch 5, wobei der erste und zweite Stoff zur Behandlung von Wunden in der Epithelisierungsphase eingesetzt wird.

**Claims**

1.  Wound system comprising

    (a) first layer containing a first substance,
    (b) a second substance disposed above or below the first layer,

    wherein the first and second substances have a different standard potential $E^0$ measured at 25°C; 101.3 kPa, pH=0, ionic activity = 1, wherein the first substance is silver and/or a silver salt, and wherein the second substance is zinc, wherein the first layer comprises a film, a foam, a fabric, a knitted fabric, a filament, a nonwoven fabric, interwoven materials and/or combinations thereof, and
    wherein the second substance is contained in a second layer, wherein the second layer comprises a polymer foam.

2.  Wound system according to claim 1, wherein the first layer comprises a polymer foam.

3.  Wound system according to claim 1 or 2, wherein the first layer comprises a polyamide knit fabric.

4.  Wound system according to claim 1, wherein the first and second layers comprise the same polymer foam.

5.  First and second substances for use in the therapeutic treatment of wounds on the human or animal body, wherein the first substance is contained in a first layer and the second substance is located above or below the first layer, and has a different standard potential $E^0$ measured at 25°C; 101.3 kPa, pH=0, ionic activity = 1, wherein the first substance is silver and/or a silver salt, and wherein the second substance is zinc, wherein the first layer comprises a film, a foam, a fabric, a knitted fabric, a filament, a nonwoven fabric, interwoven materials and/or combinations thereof, and wherein the second substance is contained in a second layer, wherein the second layer comprises a polymer foam.

6. First and second substance for use according to claim 5, wherein the first and second substances are used for treating wounds in the epithelization phase.

**Revendications**

1. Système de plaie comprenant

   (a) une première couche contenant une première substance,
   (b) une deuxième substance située au-dessus ou au-dessous de la première couche, les première et deuxième substances ayant un potentiel standard $E^0$ différent, mesuré à 25°C ; 101,3 kPa, pH = 0, activité ionique = 1, la première substance étant de l'argent et/ou un sel d'argent, et la seconde substance étant du zinc, la première couche comprenant un film, une mousse, un tissu, un ouvrage, un filament, un tissu non tissé, des matériaux entrelacés et/ou leurs combinaisons, et la deuxième substance étant contenue dans une seconde couche, la seconde couche comprenant une mousse polymère.

2. Système de plaie selon la revendication 1, la première couche comprenant une mousse polymère.

3. Système de plaie selon la revendication 1 ou 2, la première couche comprenant un tricot de polyamide.

4. Système de plaie selon la revendication 1, les première et deuxième substances comprenant la même mousse polymère.

5. Première et deuxième substances pour utilisation dans le traitement thérapeutique des plaies du corps humain ou animal, la première substance étant contenue dans une première couche et la seconde substance étant située au-dessus ou au-dessous de la première couche, et les deux ayant un potentiel standard $E^0$ différent, mesuré à 25°C ; 101,3 kPa, pH = 0, activité ionique = 1, la première substance étant de l'argent et/ou un sel d'argent, et la deuxième substance étant du zinc, la première couche comprenant un film, une mousse, un tissu, un ouvrage, un filament, un tissu non tissé, des matériaux entrelacés et/ou leurs combinaisons, et la deuxième substance étant contenue dans une seconde couche, la seconde couche comprenant une mousse polymère.

6. Première et deuxième substances pour utilisation selon la revendication 5, la première et deuxième substances étant utilisées dans le traitement de plaies dans la phase d'épithélialisation.

Figur 1

Figur 2

Figur 3

→ 33

→ 32

→ 31

Figur 3a

→ 33a

→ 32a

→ 31a

Figur 3b

→ 33b

→ 32b

→ 31b

Figur 4

42

41

Figur 4a

42a

41a

Figur 4b

43b

42b

41b

Figur 5

54

53

51

52

Figur 5a

51a

52a

53a

54a

Figur 6

→ 62

61

Figur 7

→ 71

→ 72

Figur 7a

→ 71a

72a

Figur 8

83¶

82¶

81¶

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010000451 A
- WO 2011141454 A
- EP 1715915 B1
- US 2014107740 A